# EUROPÄISCHE PATENTSCHRIFT

(11) **EP 0 652 726 B1**
(45) Veröffentlichungstag und Bekanntmachung des Hinweises auf die Patenterteilung: **16.10.1996**
(21) Anmeldenummer: 93915514.9
(22) Anmeldetag: 02.08.1993
(51) Int. Cl.: A61B 1/04, A61B 5/06

(54) **VERFAHREN ZUR DARSTELLUNG DES INNEREN VON KÖRPERN**
PROCESS FOR IMAGING THE INTERIOR OF BODIES
PROCEDE DE VISUALISATION DE L'INTERIEUR DE CORPS

(30) Priorität: 31.07.1992 AT 1557/92
(43) Veröffentlichungstag der Anmeldung: 17.05.1995
(73) Patentinhaber: TRUPPE, Michael, Dr., 1110 Wien (AT)
(72) Erfinder: TRUPPE, Michael, Dr., 1110 Wien (AT)
(74) Vertreter: Babeluk, Michael, Dipl.-Ing. Mag., Patentanwälte Babeluk - Krause
(86) Internationale Anmeldenummer: AT9300126
(87) Internationale Veröffentlichungsnummer: WO9403100

(56) Entgegenhaltungen:
- EP-A- 0 488 987
- WO-A-92/03090
- DE-A- 3 738 667

## Beschreibung

Die Erfindung betrifft ein Verfahren zur Darstellung des Inneren von Körpern, mit folgenden Schritten:
- Bereitstellung eines optischen Abbildungssystems bestehend aus einer Kamera und einem Monitor;
- Zuordnung eines räumlichen Datenfeldes zu dem in einer bestimmten Lage befindlichen Körper;
- laufende Erfassung der räumlichen Position der Kamera;
- fortgesetzte Berechnung einer Darstellung des Datenfeldes, die jeweils dem aktuellen Blickwinkel der Kamera entspricht;
- gleichzeitige oder alternative Darstellung des optischen Bildes und des Datenfeldes am Monitor.

In zunehmendem Maße wird bei Operationen ein Endoskop eingesetzt, um die Belastung für den Patienten zu verringern. Dabei wird das Endoskopbild auf einem Videomonitor dargestellt. Dies bedeutet für den Arzt eine wesentliche Änderung der Operationstechnik.

Bei einer herkömmlichen Operation ist das Operationsgebiet dem Auge frei zugänglich, und es gibt eine natürliche Koordination der Handbewegungen. Dies ist nun bei einer Operation mittels Endoskop nicht mehr gegeben. Es gibt keinen Zusammenhang zwischen der Orientierung des Endoskopes und der Blickrichtung des Anwenders, d.h. des Operateurs. Dadurch wird auch die Bewegung chirurgischer Instrumente relativ zum Endoskop abhängig vom räumlichen Vorstellungsvermögen des Arztes. Der zweite Nachteil ist die fehlende Raumempfindung, da üblicherweise nur eine Endoskoplinse verwendet wird.

Für jeden chirurgischen Eingriff ist die Kenntnis von Organ- und Tumorgrenzen sowie der anatomischen Situation im allgemeinen notwendig. Eine Übersicht über das Operationsgebiet erleichtert die Orientierung.

Der dritte Aspekt ist die Operationsplanung. Bei einem frei zugänglichen Operationsfeld ergibt sich eine klare Abfolge der Operationsschritte. Die chirurgischen Instrumente können intuitiv eingesetzt werden. Die Operation mittels Endoskop stellt größere Anforderungen. Allein die Positionierung der chirurgischen Instrumente relativ zum Operationsgebiet erfordert Planung.

Im Bereich der stereotaktischen Chirurgie gibt es Verfahren, welche im Prinzip auch für die endoskopische Chirurgie angewendet werden können.

Aus der DE-A 37 17 871 ist es bekannt, in Operationsmikroskop Daten, wie etwa Computertomographie (CT)-Darstellungen, einzublenden, um eine Hilfe bei der Navigation chirurgischer Instrumente zu erhalten. Die dargestellten CT-Schichten entsprechen der Ebene, auf die das Mikroskop fokussiert ist. Bei einer Bewegung des Instrumentes werden die entsprechenden Schichten dynamisch am Computerbildschirm dargestellt. Der Chirurg soll dadurch bei der Positionierung eines Instrumentes relativ zu einer anatomischen Struktur unterstützt werden. Um nun das Mikroskopbild mit der CT-Darstellung zur Deckung zu bringen, ist es notwendig, bestimmte Punkte, die mittels eines Laserstrahles markiert werden, mit dem Mikroskop anzuvisieren und dann das Mikroskop durchzufokussieren.

Die US-A 4,722,056 beschreibt ein Verfahren, bei dem eine Tomographiebild mit der Fokalebene eines Operationsmikroskopes überlagert wird. Die Darstellung einer CT-Schicht wird dabei der optischen Darstellung angepaßt.

Die DE-A 41 34 481 betrifft ein Mikroskop tur die stereotaktische Mikrochirurgie. Zur Bestimmung der Position des Mikroskops relativ zum Patienten wird ein Laserortungssystem verwendet. Die Funktion ist ähnlich der des Mikroskops, das in der US-A 4,722,056 beschrieben ist.

Weiters ist in der EP-A 488 987 des Anmelders ein Verfahren zur Überlagerung von Daten und optischen Darstellungen beschrieben. Mit diesem Verfahren ist es beispielsweise möglich, virtuelle Achsen von Extremitäten in Echtzeit in eine optische Darstellung einzublenden.

Im Bereich der endoskopischen Chirurgie stehen vollständige CT-Befundserien nur selten zur Verfügung. Außerdem ist die räumliche Reproduzierbarkeit der Lage von anatomischen Strukturen vor allem auf den Schädel beschränkt. In der Bauchregion ist der intraoperative Zustand ohne besondere Maßnahmen nicht aus einem präoperativen CT ableitbar. Außerdem ist eine Computertomographie ein relativ aufwendiges Verfahren, das nicht immer zur Verfügung steht oder eingesetzt werden kann.

Diese bekannten Verfahren gehen davon aus, daß die Position des Patienten vor der Operation relativ zu einem raumfesten Koordinatensystem mit allen drei Freiheitsgraden eindeutig festgelegt wird. Dies kann etwa durch das Fokussieren von Markierungspunkten mit einem Operationsmikroskop erfolgen. Nach dieser Festlegung muß der Patient starr fixiert bleiben, d.h. fest am Operationstisch eingespannt sein. Die Position des Mikroskops wird bei diesen bekannten Verfahren über das Gestänge oder über Lagesensoren erfaßt, sodaß eine CT-Darstellung o.dgl. in eine Relation zur Bildebene gebracht werden kann, die eine Überlagerung dieser Darstellung mit dem optischen Bild ermöglicht.

Diese Verfahren werden vor allem im Bereich der stereotaktischen Chirurgie angewendet. Ein chirurgisches Instrument soll zum Beispiel zu einem Tumor geführt werden. Die Lage des Tumors wird aus CT-Befunden rekonstruiert. Es darf keine Veränderung in der Lage des Patienten selbst oder der Lage des Operatiosgebietes innerhalb des Patienten nach der Erfassung der Position des Patienten, d.h. insbesonders während der Operation, auftreten.

Nun ist jedoch eine vollkommen starre Lagerung eines Patienten nicht immer möglich. Außerdem treten gerade bei endoskopischen Operationen zusätzliche Schwierigkeiten auf. Das Endoskop wird durch freie Körperhöhlen zum Zielbereich bewegt. Diese sind im allgemeinen relativ flexibel und korrelieren daher selten mit CT-Befunden. Außerdem können sich die Gewebe während einer Operation oft beträchtlich verschieben, z.B. durch Entfernen von Gewebsteilen, Absaugen von Flüssigkeit usw. Dadurch deckt sich die Darstellung des Datenfeldes immer weniger mit dem optischen Bild und die gebotene Information wird zunehmend wertlos.

Außerdem ist zu beobachten, daß aufgrund der begrenzten Genauigkeit von Lagesensoren immer nur für ein bestimmtes Raumvolumen eine optimale Positionsbestimmung möglich ist. Markierungspunkte, die unter Umständen relativ weit vom Zielbereich gelegen sind, wie dies bei endoskopischen Verfahren allgemein der Fall sein wird, sind daher im Hinblick auf die erreichbare Genauigkeit nicht optimal. Schließlich tritt bei Lagesensoren eine gewisse zeitliche Drift auf, sodaß bei längeren Eingriffen unvermeidliche Abweichungen auftreten werden.

Aufgabe der Erfindung ist es, diese Nachteile zu vermeiden und ein Verfahren zu schaffen, das auch bei Verwendung eines Endoskopes eine genaue Überlagerung der optischen Darstellung mit einem Datenfeld, z.B. einer CT-Darstellung ermöglicht.

Eine weitere Aufgabe der Erfindung ist es, ein Verfahren zur Unterstützung der Navigation bei endoskopischen Eingriffen zu schaffen, das auch ohne das Vorliegen von Darstellungen aus einer Computertomographie einsetzbar ist.

Diese Aufgabe wird dadurch gelöst, daß der Kamera ein Endoskop vorgeschaltet ist, daß wiederkehrend eine Eichung durchgeführt wird, die darin besteht, daß ein oder mehrere charakteristische Punkte des Datenfeldes mit der zugehörigen optischen Darstellung am Bildschirm durch einen Eingabevorgang des Anwenders in Übereinstimmung gebracht werden.

Wesentlich an der Erfindung ist, daß Markierungspunkte wie beim Stand der Technik nur für eine allenfalls vorgesehene " Rohnavigation" benötigt werden. Dies ermöglicht ein ungefähres Anpeilen der Zielregion. Bei der eigentlichen Navigation ist der Anwender frei in der Wahl der zur Nacheichung verwendeten Punkte. Die Nacheichung kann daher auch stets in der momentan besonders interessierenden Region durchgeführt werden, wodurch die Genauigkeit in diesem Gebiet maximiert werden. kann. Im Gegensatz zu den Verfahren des Standes der Technik, bei denen gleichsam von außen nach innen gearbeitet wird, kann man den Vorgang der vorliegenden Erfindung als Vorgehen von innen nach außen bezeichnen.

Bei Verwendung neuartiger 3D-Sensoren auf Chip-Basis, die nur mehr eine Größe von etwa 1 mm² besitzen, kann eine Vielzahl solcher Sensoren am Patienten aufgebracht werden, um ein lokales Referenzkoordinatensystem zu schaffen. Dabei können etwa 100 solcher Sensoren in der Leberregion aufgebracht werden. Die erfindungsgemäße Nacheichung wird dann mit der Positionsbestimmung durch die Referenzsensoren in Bezug gesetzt.

Als Datenfeld kann eine aus einem bildgebenden Verfahren gewonnene Darstellung, wie eine Röntgentomographie, eine Kernspintomographie, eine Ultraschalldarstellung o. dgl. verwendet werden. Um zu Darstellungen zu gelangen, die noch anschaulicher sind, als herkömmliche Schnittdarstellungen, können die CT-Befunde nachbearbeitet werden, um charakteristische Punkte oder Linien, die zum Vergleich und zum erneuten Auffinden besonders gut geeignet sind, zu erhalten. Ein solches Verfahren ist beispielsweise von N. Ayache et al.: "Evaluating 3D Registration of CT-Scan Images Using Crest Lines", in: SPIE Vol. 2035 Mathemathical in Medical Imaging II (1993), S. 60, beschrieben worden.

Besonders vorteilhaft ist es jedoch, wenn als Datenfeld eine dreidimensionale Rekonstruktion verwendet wird, die aus zuvor aufgenommenen Videoaufnahmen gewonnen wird. Damit ist es möglich, eine Navigationshilfe im Sinne der Erfindung zur Verfügung zu stellen, ohne daß die Notwendigkeit besteht, eine CT-Darstellung anfertigen zu müssen. Es wird dabei entweder vor der Operation oder in einem frühen Stadium der Operation eine lokale 3D-Rekonstruktion des Operationsgebietes erstellt. Dies ermöglicht eine genaue Operationsplanung. Nach der Durchführung von Veränderungen im Operationsgebiet z.B. durch Resektion von Gewebsteilen, Tumoren etc. kann dann die Darstellung des zuvor bestehenden Zustandes mit dem aktuellen Zustand überlagert werden.

Es ist möglich, daß die dreidimensionale Rekonstruktion aus einer einzelnen Videoaufnahme gewonnen wird, der eine Distanzmessung, z.B. über Ultraschall zugeordnet ist.

Andererseits kann die dreidimensionale Rekonstruktion aus mehreren Videoaufnahmen durch stereometrische Analyse gewonnen werden. Eine solche stereometrische Analyse ist etwa aus P. Haigron,: "3D Surface Reconstruction Using Strongly Distorted Stereo Images", in: IEEE, Proceedings of the Annual Conference on Engineering in Medicine and Biology, (1991), IEEE cat. n. 91CH3068-4, S. 1050f. bekannt. In diesem Paper wird die Rekonstruktion der Oberfläche des Femur im Kniebereich durch verzerrte Endoskopaufnahmen beschrieben. Allgemein wird die räumliche Rekonstruktion aus Einzelaufnahmen von Fua. P.: "Combining Stereo, Shading and Geometric Constraints for Surface Reconstruction from Multiple Views", in: SPIE Vol. 2031 Geometric Methods in Computer Vision II (1993), S. 112ff beschrieben.

Es ist vorteilhaft, wenn die Verschiebung der Punkte mittels einer Computermaus erfolgt. Es ist mit dem Endoskop, das in einer Körperhöhle eingeführt ist, nicht immer möglich bestimmte Punkte, die zur Nacheichung dienen sollen so anzuvisieren, daß sie genau im Fadenkreuz zu liegen kommen. Wesentlich einfacher ist es die interessierenden Punkte lediglich ins Blickfeld des Endoskops zu bringen, dann das Bild festzuhalten, d.h. einzufrieren und dann am Bildschirm die Übereinstimmung durchzuführen. Dabei können auch mehrere Punkte gleichzeitig bearbeitet werden.

Es kann weiters vorgesehen sein, daß das Endoskop zur Untersuchung eines Patienten eingesetzt wird, an dem ein Lagesensor befestigt ist, um Lageänderungen des Patienten zu kompensieren. Diese Maßnahme ermöglicht es, den Patienten auch während der Arbeit mit dem Endoskop zu bewegen. Wenn sich dabei das Koordinatensystem des Zielbereiches relativ zum Koordinatensystem des Gesamtpatienten nicht verschiebt, ist keine Nacheichung notwendig.

Weiters betrifft die Erfindung eine Vorrichtung zur Durchführung des obigen Verfahrens. Die Vorrichtung besteht aus folgenden Elementen:
- einer Kamera mit einem daran befestigten Endoskop;
- einem an der Kamera bzw. dem Endoskop befestigten Lagesensor;
- einem Monitor zur Darstellung des von der Kamera aufgenommenen optischen Bildes zusammen mit einem Datenfeld;
- einem Computer mit einem Speicher für das Datenfeld und Mitteln zur Erfassung der Position des Lagesensors;
- Mitteln zur Bestimmung der räumlichen Lage des Körpers;

Die Vorrichtung ist dadurch gekennzeichnet, daß Mittel vorgesehen sind, die es dem Benutzer ermöglichen, Punkte des Datenfeldes mit entsprechenden Punkten des optischen Bildes in Übereinstimmung zu bringen und damit die Übereinstimmung zwischen der übrigen Darstellung des Datenfeldes mit dem optischen Bild zu verbessern. Diese Mittel bestehen beispielsweise aus einer Maus, wie sie als Eingabemedium für Computer häufig eingesetzt wird und aus entsprechenden Algorithmen für die Nachjustierung der Koordinatentransformationen, um aus der Eingabe des Benutzers einen besseren "fit" zwischen der optischen Darstellung und der Darstellung des Datenfeldes zu gewinnen.

In der Folge wird die Erfindung anhand der in den Figuren dargestellten Ausführungsbeispiele näher erläutert. Es zeigen:
- Fig. 1: schematisch die Darstellung eines Endoskopbildes am Monitor vor der Eichung,
- Fig. 2: eine entsprechende Darstellung nach der Eichung,
- Fig. 3: ein Testbild zur Entzerrung des Endoskopbildes,
- Fig. 4: die durch das Endoskop verzerrte Darstellung des Testbildes,
- Fig. 5: schematisch das an einer Videokamera befestigte Endoskop,
- Fig. 6: schematisch ein Referenzobjekt zur Bestimmung räumlichen Position der Bildebene,
- Fig. 7: ein Bildschirmdarstellung zum Einmessen der Bildebene der Kamera,
- Fig. 8: schematisch die verschiedenen Koordinatensysteme und ihre Beziehungen, samt der beteiligten Hardware.

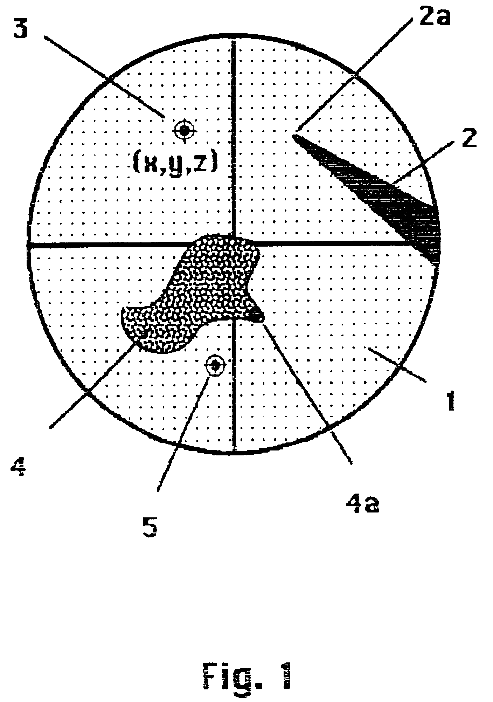

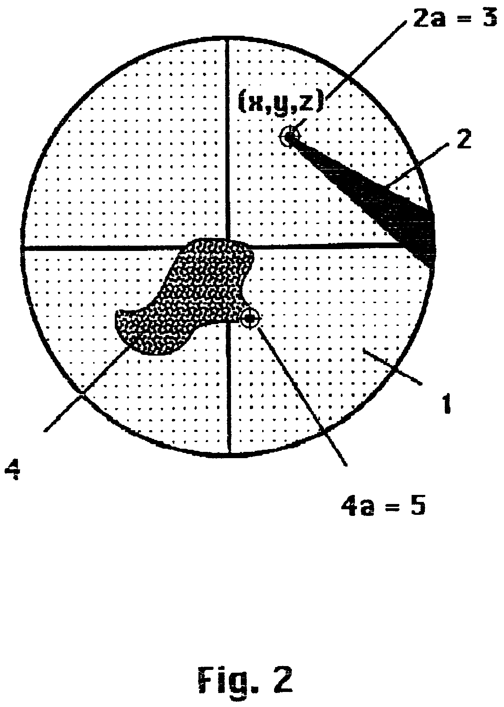

In dem Bildausschnitt 1 ist ein Instrument 2 mit einer Spitze 2a zu sehen. der Punkt 3 stellt die eingeblendete errechnete Position der Spitze dar, d.h. das "virtuelle" Bild der Spitze. In der Fig. 1 fallen das echte Bild 2a und das virtuelle Bild 3 auseinander. Durch entsprechende Justierungen sind die Bilder in Übereinstimmung zu bringen, wie dies in der Fig. 2 dargestellt ist. Damit ist die Eichung durchgeführt.

In gleicher Weise kann die Eichung mit einem charakteristischen Punkt 4a eines dargestellten Objektes 4 erfolgen. In der Fig. 1 fallen die optische Darstellung 4a und das virtuelle Bild 5 auseinander. Nach der Eichung ist dies nicht mehr der Fall.

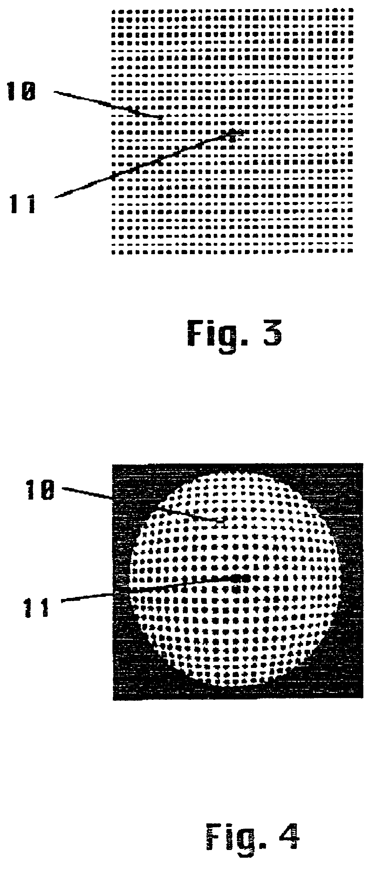

Das in der Fig. 3 dargestellte Testbild besteht aus regelmäßig in einem quadratischen Muster angeordneten Punkten 10. Durch die Optik des Endoskopes wird dieses Bild verzerrt, wie dies in der Fig. 4 dargestellt ist. Eine entsprechende Überlagerung von anderen Darstellungen ist somit mit Fehlern behaftet, die umso größer sind, je weiter sich das betreffende Detail außerhalb des Zentrums 11 befindet. Um die Übereinstimmung zu verbessern wird von einem entsprechenden Bildverarbeitungsprogramm zunächst die verzerrte Position der einzelnen Meßpunkte 10 bestimmt. Da die wahre Position bis auf einen vom Anwender bestimmbaren Skalierungsfaktor bekannt ist, kann somit für die gesamte Bildebene eine Verzerrungsfunktion berechnet werden. Mit mathematischen Methoden, die dem Fachmann geläufig sind, kann durch Invertierung dieser Funktion eine Entzerrungsfunktion errechnet werden, die die Verzerrung beseitigt. Es ist klar, daß dieser Vorgang für jedes Endoskop durchgeführt werden muß, da auch Endoskope gleicher Type durchaus unterschiedliche Verzerrungen aufweisen können.

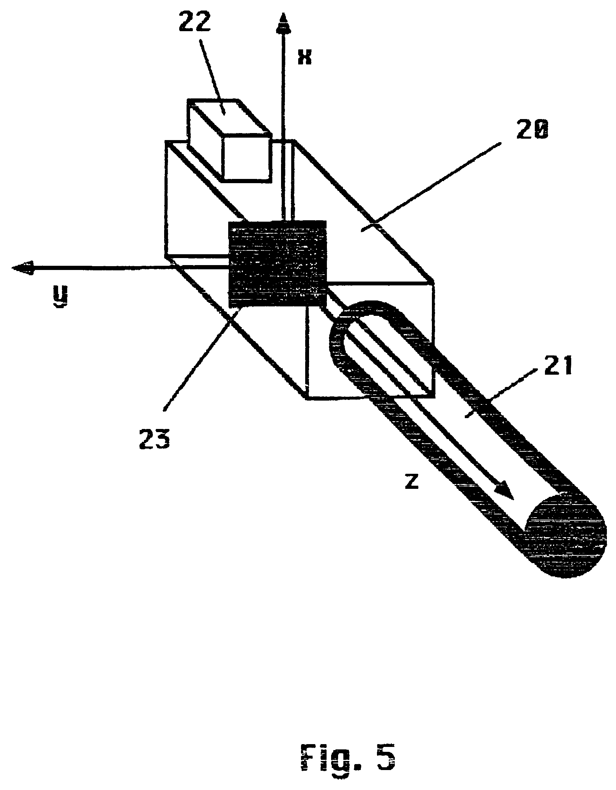

Die Fig. 5 zeigt eine Videokamera 20 mit einem an ihr befestigten Endoskop 21. Die jeweilige Lage der Kamera 20 und damit des Endoskopes 21 wird über einen 3D-Sensor 22 bestimmt. Die Bildebene der Kamera ist mit 23 bezeichnet.

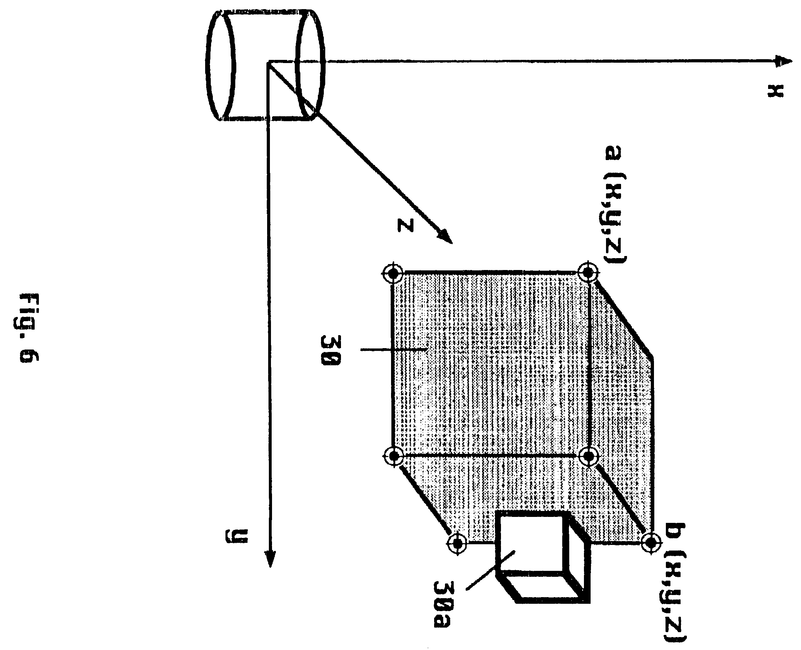

Die Fig. 6 zeigt schematisch ein festes Referenzobjekt 30 in Form eines Würfels zur Bestimmung der Position der Bildebene der Kamera. Die Koordinaten x, y, z der Eckpunkte a, b des Würfels in einem raumfesten Koordinatensystem sind bekannt. Entweder erfaßt man die Koordinaten eines im Raum unbeweglichen Würfels 30 mit dem 3D-Digitizer oder es ist, wie in der Fig. 6 dargestellt ein Lagesensor 30 an dem Würfel 30 angebracht, wodurch dieser Würfel 30 auch während der Positionsbestimmung frei im Raum beweglich sein kann. In einer am Bildschirm dargestellten symbolischen Zeichnung dieses Würfels 30 müssen vom Anwender die Eckpunkte durch Verschiebung mit der eingefrorenen Bilddarstellung in Übereinstimmung gebracht werden. Aus dieser Information ist es für den Computer möglich mit Hilfe einer direkten linearen Transformation die zum Zeitpunkt des Einfrierens des Bildes vor liegenden Koordinaten der Bildebene zu berechnen. Durch den an der Kamera 20 befestigten 3D-Sensor 22 kann auch die in der Zwischenzeit möglicherweise veränderte Position der Bildebene berechnet werden.

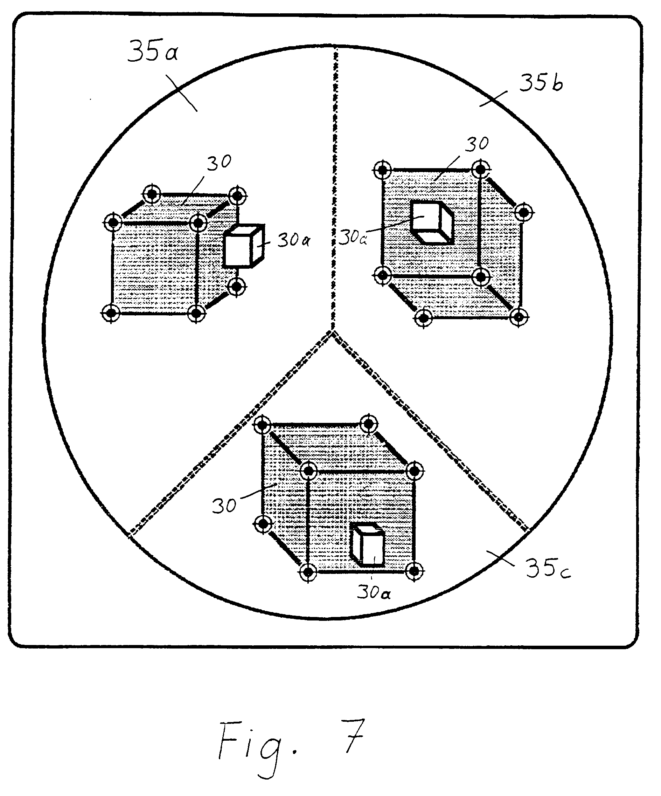

Die Fig. 7 zeigt einen Bildausschnitt, der bei der Einmessung der Position der Bildebene verwendet werden kann. In drei Sektoren 35a, 35b und 35c werden aus unterschiedlichen Positionen aufgenommene Darstellungen des Referenzobjektes, nämlich des Würfels 30, dargestellt. Dadurch ist es möglich, an einer einzigen Bildschirmdarstellung eine Vielzahl von Eichmessungen durchzuführen, um die Genauigkeit zu maximieren.

Alternativ zu einem real existierenden Referenzobjekt kann zum Einmessen des Endoskops auch ein "virtuelles Referenzobjekt" verwendet werden. Dabei wird die Kamera 20 auf die Spitze eines 3D-Stylus gerichtet. Ein 3D-Stylus ist ein Gerät in der Größe eine Kugelschreibers, das über eingebaute Magnetspulen zu jedem Zeitpunkt Daten über die räumliche Position seiner Spitze abgeben kann. Das Einmessen des Endoskops erfolgt in der Weise, daß die Kamera auf den 3D-Stylus gerichtet wird. Dann wird das Bild festgehalten, d.h. eingefroren und ein am Bildschirm befindlicher Cursor wird mit einer Maus oder einem Joy-Stick auf die Darstellung der Spitze bewegt. Dieser Vorgang wird mindestens sechs Mal durchgeführt. Auch auf diese Weise ist ein genaues Einmessen des Endoskops möglich.

Die Genauigkeit der Überlagerung kann in sehr einfacher und anschaulicher Weise dadurch erfolgen, daß der 3D-Stylus in das Bild gebracht wird. Die optische Darstellung der Spitze und die symbolische Anzeige, die aus den Koordinaten gewonnen wird, müssen bei optimaler Anpassung genau übereinander liegen. Ein Abstand zeigt eine noch nicht exakte Anpassung der Koordinatensysteme an.

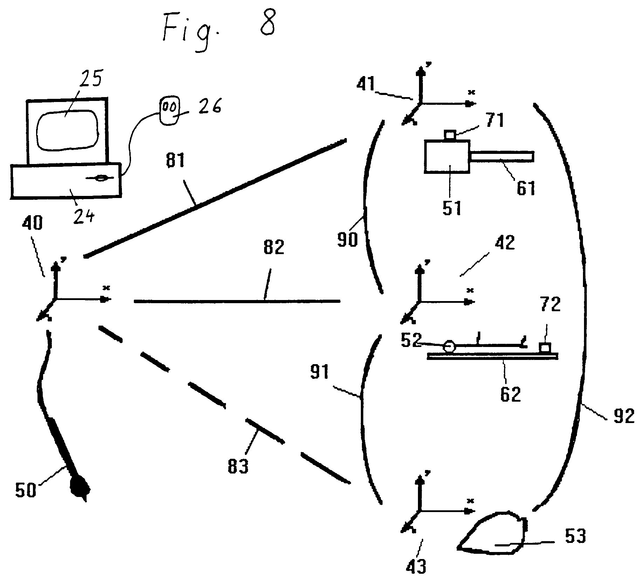

Aus der Fig. 8 ist schematisch das raumfeste Koordinatensystem 40 ersichtlich. Es wird beispielhaft durch den Digitizer-Stylus 50 repräsentiert, mit dem die Koordinaten jedes Raumpunktes durch Abtastung bestimmt werden können. Das Koordinatensystem 41 ist das des Endoskopes 61, bzw. der fest daran befestigten Kamera 51. Die laufende Position wird über den fest angebrachten Lagesensor 71 erfaßt. Die Einmessung der Bildebene erfolgt einmalig über ein Referenzobjekt oder ein virtuelles Referenzobjekt, wie oben beschrieben. Dadurch wird die Beziehung 81 zwischen den Koordinatensystemen 40 und 41 für die Dauer der endoskopischen Untersuchung festgelegt.

Am Patienten 52, der etwa auf einem Operationstisch 62 liegt, kann ein Lagesensor 72 angebracht sein. Durch eine einmalige Justierung wird die Beziehung 82 zum raumfesten Koordinatensystem festgelegt, und somit auch die Relation 90 bestimmt.

Der Zielbereich ist mit 53 angedeutet. Sein Koordinatensystem, das auch das der Datenstruktur ist, kann zunächst grob durch Einrichtung aufgrund äußerer Gegebenheiten erfaßt werden. Eine direkte Verfolgung ist nicht möglich, weshalb die Relation 83 unterbrochen dargestellt ist. Durch das erfindungsgemäße Verfahren kann jedoch die Relation 92 zur Kamera 51 bzw. zum Endoskop 61 hergestellt werden, wodurch auch die Relation 91 bestimmt ist. Wenn sich nun die Relation 91 ändert, z.B. nach der Entfernung von Gewebsteilen, so muß eine Nacheichung mit dem Verfahren der Erfindung durchgeführt werden.

Am Computer 24 werden die notwendigen Berechnungen durchgeführt und am Monitor 25 dargestellt. Die Maus 26 dient zur Durchführung der Nacheichung.

## Patentansprüche

1. Verfahren zur Darstellung des Inneren von Körpern, mit folgenden Schritten:
- Bereitstellung eines optischen Abbildungssystems bestehend aus einer Kamera (20) und einem Monitor (25);
- Zuordnung eines räumlichen Datenfeldes zu dem in einer bestimmten Lage befindlichen Körper;
- laufende Erfassung der räumlichen Position der Kamera (20);
- fortgesetzte Berechnung einer Darstellung des Datenfeldes, die jeweils dem aktuellen Blickwinkel der Kamera entspricht;
- gleichzeitige oder alternative Darstellung des optischen Bildes und des Datenfeldes am Monitor (25);
dadurch gekennzeichnet, daß der Kamera (20) ein Endoskop (21) vorgeschaltet ist, daß wiederkehrend eine Eichung durchgeführt wird, die darin besteht, daß ein oder mehrere charakteristische Punkte des Datenfeldes mit der zugehörigen optischen Darstellung am Bildschirm durch einen Eingabevorgang des Anwenders in Übereinstimmung gebracht werden.

2. Verfahren nach Anspruch 1, dadurch gekennzeichnet, daß als Datenfeld eine aus einem bildgebenden Verfahren gewonnene Darstellung, wie eine Röntgentomographie, eine Kernspintomographie, eine Ultraschalldarstellung o. dgl. verwendet wird.

3. Verfahren nach Anspruch 1, dadurch gekennzeichnet, daß als Datenfeld eine dreidimensionale Rekonstruktion verwendet wird, die aus zuvor aufgenommenen Videoaufnahmen gewonnen wird.

4. Verfahren nach Anspruch 3, dadurch gekennzeichnet, daß die dreidimensionale Rekonstruktion aus einer einzelnen Videoaufnahme gewonnen wird, der eine Distanzmessung, z.B. über Ultraschall zugeordnet ist.

5. Verfahren nach Anspruch 3, dadurch gekennzeichnet, daß die dreidimensionale Rekonstruktion aus mehreren Videoaufnahmen durch stereometrische Analyse gewonnen wird.

6. Verfahren nach einem der Ansprüche 1 bis 5, dadurch gekennzeichnet, daß der Eingabevorgang darin besteht, daß die kombinierte Darstellung des optischen Bildes mit dem Datenfeld zu einem vom Anwender bestimmten Zeitpunkt eingefroren wird und daß in dieser festgehaltenen Darstellung einzelne Punkte des Datenfeldes am Bildschirm verschoben werden können.

7. Verfahren nach Anspruch 6, dadurch gekennzeichnet, daß die Verschiebung der Punkte mittels einer Computermaus erfolgt.

8. Verfahren nach einem der Ansprüche 1 bis 7, dadurch gekennzeichnet, daß eine elektronische Entzerrung des optischen Systems des Endoskopes (21) durchgeführt wird, indem das Endoskop auf ein Testbild gerichtet wird, das aus regelmäßig angeordneten Punkten oder Linien besteht, daß eine Auswertung mit einem Mustererkennungsverfahren erfolgt und daß aus den so gewonnenen Daten eine Entzerrungsfunktion für die Optik des Endoskopes errechnet wird.

9. Verfahren nach einem der Ansprüche 1 bis 8, dadurch gekennzeichnet, daß das Endoskop zur Untersuchung eines Patienten eingesetzt wird, an dem ein Lagesensor befestigt ist, um Lageänderungen des Patienten zu kompensieren.

10. Vorrichtung zur Durchführung eines Verfahrens nach einem der Ansprüche 1 bis 9, mit folgenden Elementen:
Einer Kamera (20) mit einem daran befestigten Endoskop (21);
einem an der Kamera (20) bzw. dem Endoskop (21) befestigten Lagesensor (22);
einem Monitor (25) zur Darstellung des von der Kamera (20) aufgenommenen optischen Bildes zusammen mit einem räumlichen Datenfeld;
einem Computer (24) mit einem Speicher für das Datenfeld und Mitteln zur Erfassung der Position des Lagesensors (22);
Mitteln zur Bestimmung der räumlichen Lage des Körpers;
dadurch gekennzeichnet, daß Mittel vorgesehen sind, die es dem Benutzer ermöglichen, Punkte des Datenfeldes mit entsprechenden Punkten des optischen Bildes durch Verschiebung von mindestens einem Punkt des Datenfeldes am Bildschirm in Übereinstimmung zu bringen und damit die Übereinstimmung zwischen der übrigen Darstellung des Datenfeldes mit dem optischen Bild zu verbessern.

11. Vorrichtung nach Anspruch 10, dadurch gekennzeichnet, daß ein weiterer Lagesensor (72) vorgesehen ist, der fest an einem zu untersuchenden Patienten angebracht ist.

## Claims

1. Method for the representation of the interior of bodies, having the following steps:
- preparation of an optical imaging system comprising a camera (20) and a monitor (25);
- association of a spatial data field with the body which is in a specific location;
- continuous detection of the spatial position of the camera (20);
- continual computation of a representation of the data field that corresponds to the respective current viewing angle of the camera;
- simultaneous or alternative representation of the optical image and of the data field on the monitor (25);
characterised in that an endoscope (21) is connected in relation to the camera (20), in that calibration is carried out repeatedly, the calibration consisting in bringing one or more characteristic dots of the data field into line with the associated optical representation on the screen by means of an inputting process carried out by the user.

2. Method according to claim 1, characterised in that a representation obtained from an image-forming method, such as X-ray tomography, nuclear spin tomography, ultrasonic representation or the like, is used as the data field.

3. Method according to claim 1, characterised in that a three-dimensional reconstruction, which is obtained from video images taken previously, is used as the data field.

4. Method according to claim 3, characterised in that the three-dimensional reconstruction is obtained from one single video image, associated with which there is a distance measurement, for example, using ultrasound.

5. Method according to claim 3, characterised in that the three-dimensional reconstruction consisting of a plurality of video images is obtained by means of stereometric analysis.

6. Method according to one of the claims 1 to 5, characterised in that the inputting process consists in freezing the combined representation of the optical image and the data field at an instant which is determined by the user and in that individual dots of the data field can be displaced on the screen in this representation which is held fast.

7. Method according to claim 6, characterised in that the displacement of the dots is effected by means of a computer mouse.

8. Method according to one of the claims 1 to 7, characterised in that electronic elimination of distortion of the optical system of the endoscope (21) is effected by directing the endoscope at a test image which consists of regularly arranged dots or lines, in that an evaluation is carried out with a pattern-identification procedure and in that a distortion-elimination function for the optical system of the endoscope is calculated from the data thus obtained.

9. Method according to one of the claims 1 to 8, characterised in that the endoscope is used to examine a patient to whom a location sensor is secured in order to compensate for changes in location of the patient.

10. Arrangement for carrying out a method according to one of the claims 1 to 9, having the following elements:
- a camera (20) having an endoscope (21) secured thereto;
- a location sensor (22) secured to the camera (20) or to the endoscope (21) respectively;
- a monitor (25) for the representation of the optical image, taken by the camera (20), together with a spatial data field;
- a computer (24) having a memory for the data field and means for detecting the position of the location sensor (22);
- means for determining the location of the body in space;
characterised in that means are provided that make it possible for the user to bring dots of the data field into line with corresponding dots of the optical image by displacement of at least one dot of the data field on the screen and thus to improve the correspondence between the rest of the representation of the data field and the optical image.

11. Arrangement according to claim 10, characterised in that a further location sensor (72) is provided that is fixedly attached to a patient who is to be examined.

## Revendications

1. Procédé de visualisation de l'intérieur de corps, comportant les étapes ci-après :
- mise à disposition d'un système de représentation optique constitué d'une caméra (20) et d'un moniteur (25);
- association d'un champ de données spatial au corps se trouvant dans une position déterminée;
- appréhension en continu de la position spatiale de la caméra (20);
- calcul en continu d'une représentation du champ de données, correspondant respectivement à l'angle d'observation actuel de la caméra;
- visualisation simultanée ou alternée de l'image optique et du champ de données sur le moniteur (25);
caractérisé en ce qu'en amont de la caméra (20) est mis en circuit un endoscope (21), en ce qu'est effectué périodiquement un étalonnage consistant à placer en coïncidence un ou plusieurs points caractéristiques du champ de données avec la visualisation optique afférente sur l'écran, par un processus d'introduction de l'utilisateur.

2. Procédé selon la revendication 1, caractérisé en ce qu'on utilise comme champ de données une visualisation, obtenue d'après un procédé fournissant une image, telle qu'une tomographie aux rayons X, une tomographie à spin nucléaire, une représentation par ultrasons ou analogue.

3. Procédé selon la revendication 1, caractérisé en ce que l'on utilise comme champ de données une reconstruction tridimensionnelle ayant été obtenue d'après des clichés vidéo enregistrés au préalable.

4. Procédé selon la revendication 3, caractérisé en ce que la reconstruction tridimensionnelle est obtenue à partir d'un enregistrement vidéo individuel, auquel est associée une mesure de distance, par exemple faite par l'intermédiaire d'ultrasons.

5. Procédé selon la revendication 3, caractérisé en ce que la reconstruction tridimensionnelle est obtenue à partir d'une pluralité d'enregistrements vidéo, par une analyse stéréométrique.

6. Procédé selon l'une des revendications 1 à 5, caractérisé en ce que le processus d'introduction consiste en le fait de figer la représentation combinée de l'image optique avec le champ de données, a un moment déterminé par l'utilisateur et en ce que dans cette représentation fixée, il est possible de décaler des points individuels du champ de données, sur l'écran.

7. Procédé selon la revendication 6, caractérisé en ce que le décalage des points s'effectue au moyen d'une souris pour ordinateur.

8. Procédé selon l'une des revendications 1 à 7, caractérisé en ce qu'est effectuée une suppression électronique de la distorsion du système optique de l'endoscope (21), en dirigeant l'endoscope sur une image test, constituée de points ou de lignes disposés régulièrement, en effectuant une évaluation à l'aide d'un procédé d'identification de motif et une fonction de suppression de la distorsion de l'optique de l'endoscope étant calculée à partir des données ainsi obtenues.

9. Procédé selon l'une des revendications 1 à 8, caractérisé en ce que l'endoscope est mis en oeuvre pour l'examen d'un patient sur lequel un capteur de position est fixé afin d'obtenir une compensation des modifications de position du patient.

10. Dispositif de mise en oeuvre d'un procédé selon l'une des revendications 1 a 9, comportant les éléments ci-après :
- une caméra (20) avec un endoscope (21) fixé dessus;
- un capteur de position (22) fixé sur la caméra (20), respectivement sur l'endoscope (21);
- un moniteur (25) pour la visualisation de l'image optique enregistrée par la caméra (20), conjointement avec un champ de données spatial;
- un ordinateur (24), ayant une mémoire pour le champ de données et des moyens pour appréhender la position du capteur de position (22);
- des moyens de détermination de la position spatiale du corps;
caractérisé en ce que sont prévus des moyens permettant a l'utilisateur de placer sur l'écran des points du champ de données en coïncidence avec des points correspondants de l'image optique, par décalage d'au moins un point du champ de données et d'améliorer ainsi la coïncidence entre le reste de la visualisation du champ de données avec l'image optique.

11. Dispositif selon la revendication 10, caractérisé en ce qu'un capteur de position (72) supplémentaire est prévu, monté en position fixe sur un patient à examiner.
